# EUROPEAN PATENT SPECIFICATION

(11) **EP 0 788 812 B1**
(45) Date of publication and mention of the grant of the patent: **12.05.2004**
(21) Application number: 97850023.9
(22) Date of filing: 12.02.1997
(51) Int. Cl.: A61N 1/365, A61N 1/368, A61N 1/05

(54) **Pacemaker system comprising a bipolar sensor electrode**
Herzschrittmacher mit einer bipolaren Sensorelektrode
Système de stimulation cardiaque comprenant une électrode bipolaire de capteur

(30) Priority: 12.02.1996 SE 9600511
(43) Date of publication of application: 13.08.1997
(73) Proprietor: St. Jude Medical AB, 175 84 Järfälla (SE)
(72) Inventor: Holmström, Nils, 175 49 Järfälla (SE)
(74) Representative: Kalling, Sven Owe

(56) References cited:
- US-A- 4 901 725
- US-A- 5 228 437
- US-A- 5 480 441

## Description

### Technical field

The present invention relates to a means to achieve, in a pacemaker system, an automatic adjustment of the heart frequency to the patient's need. The system is easy to implant and causes hardly any hemodynamic or physiological disturbances. The invention also relates to a pacemaker system comprising said means.

Modern pacemaker systems may today comprise single lead VDD electrodes which measure the electrical activity in both the atrium and the ventricle and trigger the pacemaker to stimulate the ventricle in synchronization with the atrium. The VDD electrode is a four connector lead having a distal tip electrode and, spaced proximal to this, a ring electrode with a larger area than the tip electrode. These two electrodes are positioned in the right ventricle. The lead also comprises two electrodes for placement in the atrium. The four electrodes can measure the electrical activity of the heart, the two electrodes in the atrium sensing the atrial activity and the two electrodes in the ventricle sensing the ventricular activity. If, as the result of the measured electrical activity, a pulse should be triggered, then this is done using the tip electrode as the stimulating electrode and using either the pacemaker housing as an indifferent electrode (the lead functioning as a unipolar electrode) or using the ring electrode as an indifferent electrode (the lead then functioning as a bipolar electrode).

Sensors may be implanted in the heart for measurement of activity dependent quantities, e.g. oxygen pressure and blood flow. The pacemaker frequency may be adjusted in accordance with the obtained sensor readings. However, a large number of leads and electrodes are needed to achieve this.

US,A,4,779,618 concerns a device for the physiological frequency control of a heart pacemaker. This system comprises three or four electrodes in the body, i.e. the stimulation electrode, a counter electrode, a sensor electrode and possibly also a reference electrode. Both the stimulating electrode and the oxygen measuring electrode are loaded in parallel by the cathodic stimulating pulses. Thereafter the voltage between the stimulating electrode and the oxygen sensor or between the sensor electrode and the reference electrode is measured. From this measured potential the pulse rate is calculated.

US-A-5 480 441 describes a rate-responsive pacemaker pulse generator and lead system. There are two basic electrical configurations for the pacing leads, i.e. a unipolar electrode system including a tip electrode 22 referenced to a can or case electrode 24 and a bipolar lead system having a tip electrode 22 with a ring electrode 21 which is used a the reference electrode for either sensing or pacing. Thus, the pulses are measured between either the ring and the tip electrodes or between the tip and the housing. The pacemaker comprises a FDC sense amplifier which uses a circuitry to monitor the current through a virtual load connected across the sensing electrodes.

### Background of the Invention

The problem of automatically adjusting the heart frequency using as few leads and electrodes as possible is at present solved by choosing an activity sensor parameter that is built-in in the pacemaker capsule, e.g. a piezo electrical crystal. No appliance on the lead is needed in this solution, only electrodes for p-wave detection and ventricular stimulation (stimulation electrode and possibly indifferent electrode). However, this system does not work for those sensors which it is desired to place in the blood flow or within the heart. Therefore, in such cases an extra sensor lead or a single lead comprising three or more conductors is used and this may cause problems, e.g. disturbance of the blood flow or the hemodynamics.

### Brief description of the Invention

The purpose of the invention is to obtain a means by which a pacemaker system automatically adjusts a stimulation pulse parameter to the patient's need. The system should be easy to implant and cause the least possible disturbance to the hemodynamics and physiology of the patient. Further it should comprise as few leads as possible in the heart and also use the necessary leads optimally to get the best possible function.

This purpose is attained by a system where the same bipolar lead is used both as a detector of heart, for instance atrial, electrical activity (IECG) and as a physical activity sensor. Thereby it may be possible to obtain bipolar detection of both atrial IECG and ventricular IECG. It is possible to achieve unipolar stimulation of the ventricle and to detect one or more physiological activity quantities on the same bipolar lead.

The bipolar lead comprises a tip stimulation electrode and proximal to this a ring electrode functioning both as electrode for p-wave detection and as activity sensor measuring e.g. oxygen pressure. The ring electrode may in the case of an oxygen pressure sensor be made of gold or smooth vitreous carbon.

The pacemaker system of the invention is defined in claim 1.

According to one embodiment, the electrodes are arranged at such a distance from each other that when the tip electrode is introduced into the apex of the ventricle, the ring electrode will be positioned in the atrium or somewhere else where the atrial activity can be detected and a measurement of a physiological activity quantity in mixed venous blood may be obtained (e.g. the opening to vena cava). The distance between the electrodes may be 6-15 cm, for instance 8-15 cm, preferably 10-12 cm.

According to another embodiment the electrodes are arranged at such a distance between each other that when the tip electrode is introduced into the apex of the ventricle, then the ring electrode will be also be positioned in the ventricle. In this case the distance between the electrodes may be 5-30 mm, preferably 10-20 mm.

Thus, the invention concerns a pacemaker system comprising an electrode lead having a distal tip electrode for supplying stimulation pulses to heart tissue and, proximal thereto, a ring electrode, said electrode lead being attached to a heart stimulator, said heart stimulator comprising detecting means connected to said electrode lead for detecting electrical heart activity by measurement between said tip electrode and said ring electrode, wherein said electrode lead comprises only said tip and ring electrodes, said ring electrode being a sensor of at least one physical activity dependent function and said heart stimulator comprising means for applying a measuring parameter on said ring electrode during a specified measuring period and means for measuring a measurement value through said ring electrode during at least part of said period, said heart stimulator also comprising means for adjusting, dependent on said measured measurement value, a stimulation pulse parameter.

The measuring parameter may for instance be a potential or a voltage and the corresponding measurement value a current. Another possibility is to use a current as the measuring parameter, the corresponding measurement value being a potential or a voltage. The stimulation pulse parameter may for instance be the stimulation frequency or the A-V interval

According to a preferred embodiment of the invention the system further comprises means for controlling a stimulation pulse parameter, for instance the frequency, of a pacemaker in accordance with a physical activity, said pacemaker being adapted to be implanted in a human body and having a heart stimulator in a housing with a connector housing including an integrated reference electrode, a bipolar lead with a distal tip electrode, adapted to be implanted in a ventricle, and 6-15 cm proximal thereto a ring electrode, said system comprising means for detecting the potential difference between said tip electrode and said ring electrode, means for applying on said ring electrode, shortly after detection of a QRS wave, a physiological quantity measuring parameter in relation to said reference electrode for a short specified measuring time period, means for measuring a corresponding measurement value between said pacemaker housing and said ring electrode during at least a part of said measuring period, and means for triggering stimulating pulses between said indifferent pacemaker housing and said tip electrode at a stimulation pulse parameter dependent on the measured measurement value.

According to another embodiment of the invention the system further comprises means for controlling a stimulation pulse parameter, for instance the frequency, of a pacemaker in accordance with a physical activity, said pacemaker being adapted to be implanted in a human body and having a heart stimulator in a housing with a connector housing including an integrated reference electrode, a bipolar lead with a distal tip electrode adapted to be implanted in a ventricle, and proximal thereto a ring electrode, said system comprising means for detecting the potential difference between said tip electrode and said ring electrode, means for applying on said ring electrode, shortly after detection of a QRS wave, a first physiological quantity measuring parameter in relation to said reference electrode for a short specified measuring time period, means for measuring the corresponding measurement value between said pacemaker housing and said ring electrode during at least a part of said measuring period, means for applying a second alternating measuring parameter between said tip electrode and said housing and means for measuring the corresponding measurement value between said tip electrode and said ring electrode and means for triggering stimulating pulses between said indifferent pacemaker housing and said tip electrode at a stimulation pulse parameter dependent on both the first measured measurement value and the second measured measurement value.

### Brief description of the Drawings

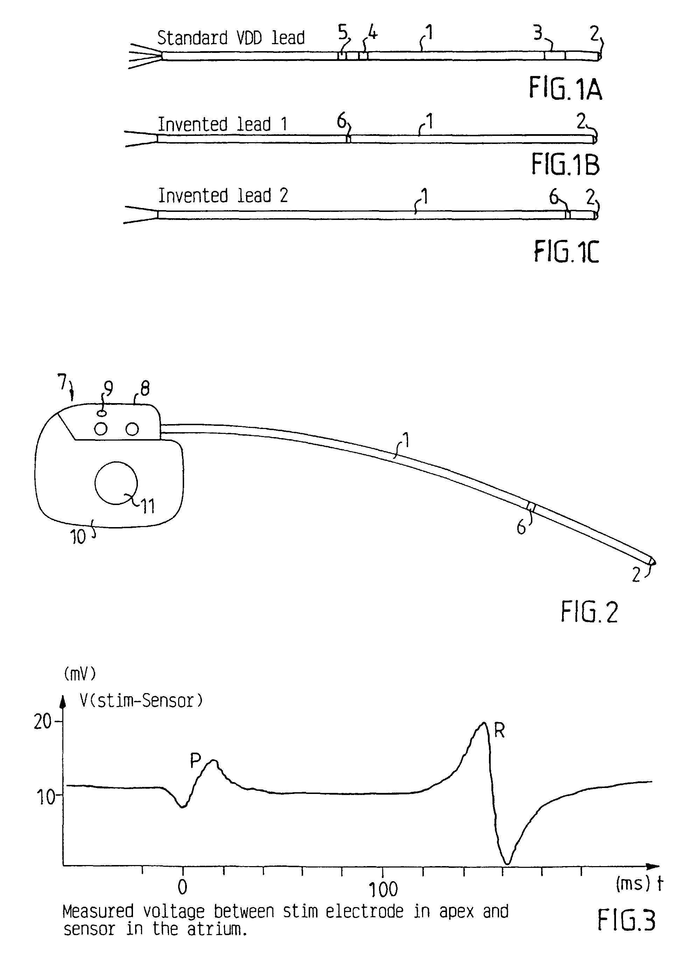

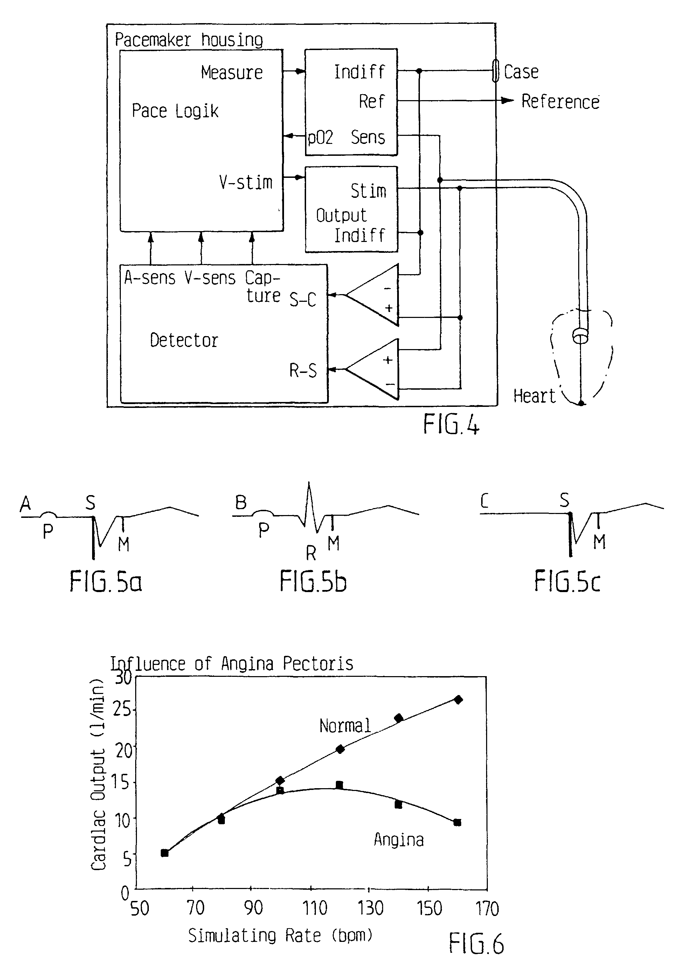

In the drawings, Fig. 1 shows a schematic view of a conventional VDD lead and of a first and a second embodiment of a lead according to the invention. Fig. 2 shows a pacemaker according to the invention and Fig. 3 is a diagram showing the measured voltage in the pacemaker in Fig. 2, between the stimulation electrode in the apex and the sensor in the atrium during a normal heartbeat. Fig. 4 is a block diagram of a pacemaker system comprising a lead according to the invention and Fig 5a-c are three different voltage measurements obtained from the system in Fig. 4. Fig. 6 is a diagram showing the change in cardiac output when the heart rate is increased, for a normal heart and for a heart having angina pectoris.

### Detailed description of the Invention

Lead A in Fig. 1 is a standard VDD lead comprising four connectors and four electrodes, the tip electrode 2, the proximal ring electrode 3, (these two to be positioned in the ventricle), and the two atrial electrodes 4 and 5. The atrial activity is measured between the electrodes 4 and 5 and the ventricular activity between the electrodes 2 and 3 or between the electrode 2 and the pacemaker housing. When an atrial depolarization is not followed by a ventricular depolarization, a stimulation pulse is delivered between the tip electrode 2 and the electrode 3 or the housing. No rate modulation depending on the physical activity of the patient is possible with this lead, except the modulation in accordance with the sinus frequency.

Lead B in Fig. 1 is an embodiment of a lead according to the invention. This lead stimulates in the ventricle, measures the heart electrical activity in both the ventricle and the atrium, in response to which it either inhibits or triggers a stimulation pulse, and is rate modulation programmable. This lead may be used in a pacemaker working in the VDDR mode. The lead according to the invention is much less complex than the former VDD lead. The new lead comprises only two connectors and two electrodes, the tip electrode 2, to be positioned in the ventricle, and the ring electrode 6, replacing the electrodes 3, 4, 5 of the lead A. The ring electrode 6 can be used to measure the atrial electrical activity as well as to sense a quantity dependent on the physical activity of the patient.

Lead C in Fig. 1 is another embodiment of a lead according to the invention. Lead C stimulates in the ventricle, measures the heart electrical activity in the ventricle, in response to which it can inhibit a stimulation pulse, and is rate modulation programmable (by a dual sensor). This new lead comprises only two connectors and two electrodes, the tip electrode 2 and the ring electrode 6, both to be placed in the ventricle. They replace the electrodes 3, 4, 5 of the lead A. The ring electrode 6 can be used to measure the ventricular electrical activity as well as to sense two parameters dependent on the physical activity of the patient. The new lead C may be used in a pacemaker system working in the VVI-2R mode.

The pacemaker system in Fig. 2 comprises a heart stimulator 7 having a housing 10 and a bipolar electrode lead 1 attached thereto according to the embodiment of the invention shown in Fig. 1B. The housing 10 has a carbon surface layer 11, used as the indifferent electrode, and an epoxy connector housing 8 with an integrated reference electrode 9. The lead 1 comprises a ring sensor electrode 6 and a tip stimulation electrode 2. The stimulation electrode is arranged at the distal end of the lead 1 to be inserted into the ventricle and is a standard stimulation electrode made of e.g. activated carbon. The sensor electrode 6 is a gold ring and may be arranged about 11 cm from the tip electrode and thus be positioned in the atrium when the tip electrode is inserted into the apex. The sensor may have an area of about 1-10 mm², e.g. about 7 mm².

The sensor electrode 6 may e.g. be an oxygen pressure sensor, an electrochemical blood flow sensor, an impedance sensor, a pH sensor, a carbondioxide pressure sensor, etc.

The sensor electrode 6 may e.g. be made of gold, vitreous carbon, graphite, pyrolytic carbon, possibly containing small amounts of silicon, deposited on a metal such as titanium, platinum, iridium or iridium oxide.

During the detection step the system monitors the voltage difference between the stimulation electrode 2 and the sensor electrode 6. The detected voltage is shown graphically in the diagram of Fig 3. By the morphology of the curve it is possible to determine whether the detected event originates from the ventricle or the atrium. A p-wave has the derivative sign (- + -) and a QRS wave the derivative sign (+ - +). Should the QRS wave fail to come after a p-wave, a stimulation pulse is delivered between the stimulation electrode 2 and the indifferent electrode on the pacemaker housing 10.

The sensor potential is floating all the time, except when the measurement pulse is delivered. The measurement pulse may be delivered during the absolute refractory time 2-100 ms, e.g. about 50 ms, after a QRS-wave or a stimulation pulse. Then the potential of the sensor 6 is forced to -0.8 V compared to the reference electrode 9 for 15 ms. At this potential the oxygen in the blood will be reduced to hydroxyl ions at the sensor 6 and an electrical current will flow between the sensor 6 and the indifferent electrode 10. This current is dependent on the venous oxygen pressure. The pacemaker rate is determined from this sensor value. Also the AV time can be optimised according to the oxygen pressure. As the oxygen pressure is always measured directly after a stimulation pulse or a QRS-wave, the measurement pulse does not disturb the p-wave or the QRS detectors. The measurement pulse may be delivered after each stimulation pulse or QRS-wave or at longer intervals, e.g. after every second, third, fourth or fifth stimulation pulse or QRS-wave, or at constant time intervals (triggered by the QRS-wave).

It is also possible with the sensor 6 to measure other quantities dependent on the physical activity. For instance, it is possible to measure the blood flow by using a flow sensor according to EP-A-620 420.

Fig. 4 is a block diagram showing a pacemaker system with a combined VDD and sensor lead. The pacemaker housing includes pace logic, a detector, a stimulation pulse output unit and an oxygen pressure measuring unit. The lead 1 comprises a tip stimulation electrode (S) and a ring sensor electrode (R). The pacemaker housing 11 ( C) serves as the indifferent electrode.

The pacemaker with a single bipolar lead shown in Fig. 4 is used for
1. Mixed venous oxygen pressure measurement in the atrium.
2. Bipolar atrial IECG sensing.
3. Bipolar and unipolar ventricular IECG sensing.
4. Ventricular capture sensing.
5. Ventricular unipolar stimulation with auto-threshold function.

The curves in Figs. 5a, 5b, 5c are surface ECG curves and illustrate three different heart condition cases.
Fig. 5a: No QRS-wave is detected. The ventricular stimulation pulse is synchronised to the p-wave. The pacemaker works like this at AV-block and functioning sinus node.
Fig. 5b: Non-pathological beats. The pacemaker just monitors the heart function. If this situation is stable the activity sensor or sensors can be programmed off.
Fig. 5c: No sinus wave is detected. During situations when the sinus rhythm cannot be used (e.g. during sinus arrest and atrial flutter) the pacemaker is working in the VVIR mode until sinus is normal. The VVIR mode pacemaker modifies the pulse rate in accordance with the oxygen pressure measured at the ring sensor electrode.
Fig. 6 shows the development of the cardiac output with increasing heart rate in a normal heart and in a heart suffering from angina pectoris.

Patients with angina pectoris get a decreased cardiac output when the heart stimulating rate is increased above a relatively low frequency a. The reason is that the myocardium becomes ischemic and the muscle stiff. The stroke volume is reduced rapidly with increased stimulating frequency.

If only the oxygen pressure controls the heart rate of these patients, a positive feedback occurs and the rate increases simultaneously as the cardiac output decreases. This is a dangerous situation for the patient which can be avoided by using the second embodiment of the present invention (Fig. 1C). The stroke volume can be measured either by sensing the blood flow or by measuring the impedance. By monitoring the flow or the impedance profile during the physical heart beat, starting directly after measurement of partial oxygen pressure (p02-measurement), morphology changes can be used to detect an ischemic heart. Then the stimulating rate can be decreased to a basic rate and the heart will have a possibility to relax.

Thus, using the lead of Fig. 1C a polarographic p02 measurement is made in the ventricle. Thereafter, the stroke volume is measured. An alternating current or voltage is applied between the tip electrode 2 and the indifferent electrode 11. The voltage between the ring electrode 6 and the stimulating electrode 2 is detected. The resulting measurement is processed to determine and monitor the impedance variations in the ventricle. During ischemia the impedance variations are smaller than normal.

The lead of Fig. 1C is also used for bipolar detection of ventricular activity in the conventional way.

## Claims

1. A pacemaker system comprising an electrode lead (1) having a distal tip electrode (2) for supplying stimulation pulses to heart tissue and, proximal thereto, a ring electrode (6), said system further comprising a heart stimulator (7) to which said electrode lead (1) is attached, said heart stimulator comprising detecting means connected to said electrode lead (1) for detecting electrical heart activity by measurement between said tip electrode (2) and said ring electrode (6), **characterized in** said heart stimulator (7) comprises a pacemaker housing (10) and a connector housing (8) with an integrated reference electrode (9), said electrode lead (1) comprising only said tip (2) and ring (6) electrodes, said ring electrode (6) being a sensor of at least one physical activity dependent function and said heart stimulator comprising means for applying a measuring parameter between said ring electrode (6) and said reference electrode (9) during a specified measuring period and means for measuring a measurement value which is a current or voltage or potential between said ring electrode (6) and said housing (10) during at least part of said period, said heart stimulator (7) also comprising means for adjusting, dependent on said measured measurement value, a stimulation pulse parameter.

2. A system according to claim 1, wherein said measuring parameter is a potential or a voltage and said corresponding measurement value is a current.

3. A system according to claim 1, wherein said measuring parameter is a current and said corresponding measurement value is a potential or a voltage.

4. A system according to any of claims 1-3, wherein said stimulation pulse parameter is the stimulation frequency or the A-V interval.

5. A system according to any of claims 1-4, wherein the distance between said tip electrode (2) and said ring electrode (6) is 6-15 cm, preferably 8-15 cm, especially 10-12 cm.

6. A system according to any of claims 1-4, wherein the distance between said tip electrode (2) and said ring electrode (6) is 5-30 mm, preferably 10-20 mm.

7. A system according to any of claims 1-6, wherein said ring electrode (6) is a sensor of one or more of: blood oxygen pressure, blood flow, impedance, pH value, carbon dioxide pressure.

8. A pacemaker system according to any of claims 1-7, comprising means for applying two or more measuring parameters between said ring electrode (6) and said reference electrode (9) during two or more consecutive predetermined measuring periods and means for measuring the corresponding measurement value between said ring electrode (6) and said housing (10) during each of said measuring periods.

9. A pacemaker system according to any of claims 1-7, comprising means for applying first a DC measuring parameter between said ring electrode (6) and said reference electrode (9) and then, during a consecutive predetermined measuring period, applying a second AC measuring parameter between said housing (10) and said tip electrode (2) and means for measuring the corresponding first measurement value between said ring electrode (6) and said housing (10) during the first of said measuring periods and means for measuring the corresponding second measurement value between said tip electrode (2) and said ring electrode (6) during the second measuring period.

10. A system according to any of claims 1-5 and 7-9, wherein said heart stimulator is arranged in a housing (10) with a connector housing (8) including an integrated reference electrode (9), said bipolar lead (1) having a distal tip electrode (2) being adapted to be implanted in a ventricle, and 6-15 cm proximal thereto a ring electrode (6), said system comprising means for detecting the potential difference between said tip electrode (2) and said ring electrode (6), means for applying on said ring electrode (6), shortly after detection of a QRS wave, a physiological quantity measuring parameter in relation to said reference electrode (9) for a short specified measuring time period, means for measuring the corresponding measurement value between said pacemaker housing (10) and said ring electrode (6) during at least a part of said measuring period, and means for triggering stimulating pulses between said indifferent pacemaker housing (10) and said tip electrode (2) at a stimulation pulse parameter dependent on the measured measurement value.

11. A system according to any of claims 1-9, wherein said heart stimulator is arranged in a housing (10) with a connector housing (8) including an integrated reference electrode (9), said bipolar lead (1) having a distal tip electrode (2) adapted to be implanted in a ventricle, and proximal thereto a ring electrode (6), said system comprising means for detecting the potential difference between said tip electrode (2) and said ring electrode (6), means for applying on said ring electrode (6), shortly after detection of a QRS wave, a first physiological quantity measuring parameter in relation to said reference electrode (9) for a short specified measuring time period, means for measuring a first corresponding measurement value between said pacemaker housing (10) and said ring electrode (6) during at least a part of said measuring period, means for applying an alternating second measuring parameter between said tip electrode (2) and said housing (10) and means for measuring a second corresponding measurement value between said tip electrode (2) and said ring electrode (6) and means for triggering stimulating pulses between said indifferent pacemaker housing (10) and said tip electrode (2) at a stimulation pulse parameter dependent on both the measured first measurement value and the second measurement value.

12. A system according to claim 10 or 11, wherein said pacemaker housing (10) has an external layer of carbon, the measurement value, preferably the current, being measured between said carbon layer and said ring electrode (6).

## Patentansprüche

1. Schrittmachersystem, enthaltend eine Elektrodenleitung (1) mit einer distalen Spitzenelektrode (2) zum Zuführen von Stimulationsimpulsen zum Herzgewebe und proximal hierzu einer Ringelektrode (6), wobei das genannte System ferner einen Herzstimulator (7) enthält, an den die genannte Elektrodenleitung (1) angebracht ist, der genannte Herzstimulator eine mit der genannten Elektrodenleitung (1) verbundene Detektionsvorrichtung enthält, zum Erfassen von elektrischer Herzaktivität durch eine Messung zwischen der genannten Spitzenelektrode (2) und der genannten Ringelektrode (6), **dadurch gekennzeichnet, dass** der genannte Herzstimulator (7) ein Schrittmachergehäuse (10) und ein Verbindergehäuse (8) mit einer integrierten Bezugselektrode (9) enthält, die genannte Elektrodenleitung (1) nur die genannte Spitzenelektrode (2) und Ringelektrode (6) enthält, die genannte Ringelektrode (6) ein Sensor von wenigstens einer physischen Aktivität abhängigen Funktion ist und der genannte Herzstimulator eine Vorrichtung enthält, zum Anbringen eines Messparameters zwischen der genannten Ringelektrode (6) und der genannten Bezugselektrode (9) während einer speziellen Messperiode und eine Vorrichtung zum Messen, während wenigstens einem Teil der genannten Periode, eines Messwertes, der ein Strom oder eine Spannung oder ein Potential zwischen der genannten Ringelektrode (6) und dem genannten Gehäuse (10) ist, ferner der Herzstimulator (7) auch eine Vorrichtung zum Einstellen eines Stimulationsimpulsparameters, abhängig von dem gemessenen Messwert, enthält.

2. System nach Anspruch 1, bei dem der genannte Messparameter ein Potential oder eine Spannung ist, und der genannte entsprechende Messwert ein Strom ist.

3. System nach Anspruch 1, bei dem der genannte Messparameter ein Strom ist und der genannte entsprechende Messwert ein Potential oder eine Spannung ist.

4. System nach einem der Ansprüche 1 bis 3, bei dem der genannte Stimulationsimpulsparameter die Stimulationsfrequenz oder das A-V-Intervall ist.

5. System nach einem der Ansprüche 1 bis 4, bei dem der Abstand zwischen der genannten Spitzenelektrode (2) und der genannten Ringelektrode (6) 6 bis 15 cm, vorzugsweise 8 bis 15 cm, insbesondere 10 bis 12 cm ist.

6. System nach einem der Ansprüche 1 bis 4, bei dem der Abstand zwischen der genannten Spitzenelektrode (2) und der genannten Ringelektrode (6) 5 bis 30 mm, vorzugsweise 10 bis 20 mm ist.

7. System nach einem der Ansprüche 1 bis 6, bei dem die genannte Ringelektrode (6) ein Sensor von einer oder mehreren der folgenden Arten ist: Blutsauerstoffdruck, Blutfluss, Impedanz, pH-Wert, Kohlendioxiddruck.

8. Schrittmachersystem nach einem der Ansprüche 1 bis 7, enthaltend eine Vorrichtung zum Anbringen von zwei oder mehr Messparametern zwischen der genannten Ringelektrode (6) und der genannten Bezugselektrode (9), während zweier oder mehrerer aufeinander folgender vorbestimmter Messperioden und eine Vorrichtung zum Messen des entsprechenden Messwertes zwischen der genannten Ringelektrode (6) und dem genannten Gehäuse (10) während jeder der genannten Messperioden.

9. Schrittmachersystem nach einem der Ansprüche 1 bis 7, enthaltend eine Vorrichtung zum Anbringen eines ersten Gleichstrommessparameters zwischen der genannten Ringelektrode (6) und der genannten Bezugselektrode (9) und dann, während einer darauffolgenden vorbestimmten Messperiode zum Anbringen eines zweiten Wechselstrommessparameters zwischen dem genannten Gehäuse (10), und der genannten Spitzenelektrode (2) und eine Vorrichtung zum Messen des entsprechenden ersten Messwertes zwischen der genannten Ringelektrode (6) und dem genannten Gehäuse (10) während der ersten der genannten Messperioden und eine Vorrichtung zum Messen des entsprechenden zweiten Messwertes zwischen der genannten Spitzenelektrode (2) und der genannten Ringelektrode (6), während der zweiten Messperiode.

10. System nach einem der Ansprüche 1 bis 5 und 7 bis 9, bei dem der genannte Herzstimulator angeordnet ist in einem Gehäuse (10) mit einem Verbindergehäuse (8), enthaltend eine integrierte Bezugselektrode (9), die genannte bipolare Leitung (1) eine distale Spitzenelektrode (2) aufweist, die ausgelegt ist, in einen Ventrikel implantiert zu werden und 6 bis 15 cm proximal hierzu eine Ringelektrode (6), das genannte System eine Vorrichtung zum Erfassen der Potentialdifferenz zwischen der genannten Spitzenelektrode (2) und der genannten Ringelektrode (6) enthält, ferner eine Vorrichtung zum Anbringen eines physiologischen Quantitätsmessparameters an die genannte Ringelektrode (6) kurz nach Detektion einer QRS-Welle in Relation zu der genannten Bezugselektrode (9) für eine kurze spezielle Messzeitperiode, eine Vorrichtung zum Messen des entsprechenden Messwertes zwischen dem genannten Schrittmachergehäuse (10) und der genannten Ringelektrode (6) während wenigstens eines Teils der genannten Messperiode, und eine Vorrichtung zum Triggern von Stimulationsimpulsen zwischen dem genannten indifferenten Schrittmachergehäuse (10) und der genannten Spitzenelektrode (2) bei einem von dem gemessenen Messwert abhängigen Stimulationsimpulsparameter.

11. System nach einem der Ansprüche 1 bis 9, bei dem der genannte Herzstimulator angeordnet ist in einem Gehäuse (10) mit einem Verbindergehäuse (8), enthaltend eine integrierte Bezugselektrode (9), die genannte bipolare Leitung (1) eine distale Spitzenelektrode (2) aufweist, die ausgelegt ist, in einen Ventrikel implantiert zu werden und eine hierzu proximale Ringelektrode (6), das genannte System eine Vorrichtung zum Detektieren der Potentialdifferenz zwischen der genannten Spitzenelektrode (2) und der genannten Ringelektrode (6) enthält, eine Vorrichtung zum Anbringen an die genannte Ringelektrode (6), kurz nach der Detektion einer QRS-Welle, einen ersten physiologischen Quantitätsmessparameter in Relation zu der genannten Bezugselektrode (9) für eine kurze spezielle Messzeitperiode, eine Vorrichtung zum Messen eines ersten entsprechenden Messwertes zwischen dem genannten Schrittmachergehäuse (10) und der genannten Ringelektrode (6), während wenigstens eines Teils der genannten Messperiode, eine Vorrichtung zum Anbringen eines wechselnden zweiten Messparameters zwischen der genannten Spitzenelektrode (2) und dem genannten Gehäuse (10) und eine Vorrichtung zum Messen eines zweiten entsprechenden Messwertes, zwischen der genannten Spitzenelektrode (2) und der genannten Ringelektrode (6) sowie eine Vorrichtung zum Triggern von Stimulationsimpulsen zwischen dem genannten indifferenten Schrittmachergehäuse (10) und der genannten Spitzenelektrode (2) bei einem Stimulationsimpulsparameter abhängig von sowohl dem gemessenen ersten Messwert als auch dem zweiten Messwert.

12. System nach Anspruch 10 oder 11, bei dem das genannte Schrittmachergehäuse (10) eine Außenschicht aus Kohlenstoff aufweist und der Messwert, vorzugsweise der Strom, zwischen der genannten Kohlenstoffschicht und der genannten Ringelektrode (6) gemessen wird.

## Revendications

1. Système formant stimulateur cardiaque comportant une dérivation (1) d'électrode ayant une électrode (2) de pointe distale pour fournir des impulsions de stimulation à du tissu cardiaque et, proximalement à celle-ci, une électrode (6) de bague, le système comportant, en outre, un stimulateur (7) cardiaque auquel la dérivation (1) d'électrode est fixée, le stimulateur cardiaque comportant des moyens de détection connectés à la dérivation (1) d'électrode pour détecter une activité cardiaque électrique par une mesure entre l'électrode (2) de pointe et l'électrode (6) de bague, **caractérisé en ce que** le stimulateur (7) cardiaque comporte un boîtier (10) de stimulateur cardiaque et un boîtier (8) de connecteur ayant une électrode (9) de référence intégrée, la dérivation (1) d'électrode comportant uniquement les électrodes (2) de pointe et (6) de bague, l'électrode (6) de bague étant un capteur d'au moins une fonction dépendant de l'activité physique et le stimulateur cardiaque comportant des moyens destinés à appliquer un paramètre de mesure entre l'électrode (6) de bague et l'électrode (9) de référence pendant une durée de mesure précisée et des moyens destinés à mesurer une valeur de mesure qui est un courant ou une tension ou un potentiel entre l'électrode (6) de bague et le boîtier (10) pendant au moins une partie de ladite durée, le stimulateur (7) cardiaque comportant également des moyens destinés à ajuster un paramètre d'impulsion de stimulation, en fonction de la valeur de mesure mesurée.

2. Système suivant la revendication 1, dans lequel le paramètre de mesure est un potentiel ou une tension et la valeur de mesure correspondante est un courant.

3. Système suivant la revendication 1, dans lequel le paramètre de mesure est un courant et la valeur de mesure correspondante est un potentiel ou une tension.

4. Système suivant l'une quelconque des revendications 1 à 3, dans lequel le paramètre d'impulsion de stimulation est la fréquence de stimulation ou l'intervalle A-V.

5. Système suivant l'une quelconque des revendications 1 à 4, dans lequel la distance entre l'électrode (2) de pointe et l'électrode (6) de bague est comprise entre 6 et 15 cm, de préférence entre 8 et 15 cm, notamment entre 10 et 12 cm.

6. Système suivant l'une quelconque des revendications 1 à 4, dans lequel la distance entre l'électrode (2) de pointe et l'électrode (6) de bague est comprise entre 5 et 30 mm, de préférence entre 10 et 20 mm.

7. Système suivant l'une quelconque des revendications 1 à 6, dans lequel l'électrode (6) de bague est un capteur de l'un ou plusieurs de : la pression de l'oxygène du sang, le débit sanguin, l'impédance, la valeur de pH, la pression du dioxyde de carbone.

8. Système formant stimulateur cardiaque suivant l'une quelconque des revendications 1 à 7, comportant des moyens destinés à appliquer deux paramètres de mesure ou plus entre l'électrode (6) de bague et l'électrode (9) de référence pendant deux durées de mesure déterminées à l'avance consécutives ou plus et des moyens destinés à mesurer la valeur de mesure correspondante entre l'électrode (6) de bague et le boîtier (10) pendant chacune des durées de mesure.

9. Système formant stimulateur cardiaque suivant l'une quelconque des revendications 1 à 7, comportant des moyens destinés à appliquer d'abord un paramètre de mesure à courant continu entre l'électrode (6) de bague et l'électrode (9) de référence, et ensuite, pendant une durée de mesure déterminée à l'avance consécutive, destinés à appliquer un deuxième paramètre de mesure à courant alternatif entre le boîtier (10) et l'électrode (2) de pointe et des moyens destinés à mesurer la première valeur de mesure correspondante entre l'électrode (6) de bague et le boîtier (10) pendant la première des durées de mesure et des moyens destinés à mesurer la deuxième valeur de mesure correspondante entre l'électrode (2) de pointe et l'électrode (6) de bague pendant la deuxième durée de mesure.

10. Système suivant l'une quelconque des revendications 1 à 5 et 7 à 9, dans lequel le stimulateur cardiaque est agencé dans un boîtier (10) ayant un boîtier (8) de connecteur comportant une électrode (9) de référence intégrée, la dérivation (1) bipolaire ayant une électrode (2) de pointe distale qui est conçue pour être implantée dans un ventricule, et de 6 à 15 cm proximalement à celle-ci, une électrode (6) de bague, le système comportant des moyens destinés à détecter la différence de potentiel entre l'électrode (2) de pointe et l'électrode (6) de bague, des moyens destinés à appliquer sur l'électrode (6) de bague, peu de temps après la détection d'une onde QRS, un paramètre de mesure d'une quantité physiologique en relation à l'électrode (9) de référence pour une durée de mesure précisée courte, des moyens destinés à mesurer la valeur de mesure correspondante entre le boîtier (10) de stimulateur et l'électrode (6) de bague pendant au moins une partie de la durée de mesure, et des moyens destinés à déclencher des impulsions de stimulation entre le boîtier (10) de stimulateur indifférent et l'électrode (2) de pointe à un paramètre d'impulsion de stimulation fonction de la valeur de mesure mesurée.

11. Système suivant l'une quelconque des revendications 1 à 9, dans lequel le stimulateur cardiaque est agencé dans un boîtier (10) ayant un boîtier (8) de connecteur comportant une électrode (9) de référence intégrée, la dérivation (1) bipolaire ayant une électrode (2) de pointe distale conçue pour être implantée dans un ventricule, et proximalement à celle-ci, une électrode (6) de bague, le système comportant des moyens destinés à détecter la différence de potentiel entre l'électrode (2) de pointe et l'électrode (6) de bague, des moyens destinés à appliquer sur l'électrode (6) de bague, peu de temps après une détection d'une onde QRS, un premier paramètre de mesure de quantité physiologique en relation à l'électrode (9) de référence pendant une durée de mesure précisée courte, des moyens destinés à mesurer une première valeur de mesure correspondante entre le boîtier (10) de stimulateur et l'électrode (6) de bague pendant au moins une partie de la durée de mesure, des moyens destinés à appliquer un deuxième paramètre de mesure alternatif entre l'électrode (2) de pointe et le boîtier (10) et des moyens destinés à mesurer une deuxième valeur de mesure correspondante entre l'électrode (2) de pointe et l'électrode (6) de bague, et des moyens destinés à déclencher des impulsions de stimulation entre le boîtier (10) de stimulateur indifférent et l'électrode (2) de pointe à un paramètre d'impulsion de stimulation qui est fonction à la fois de la première valeur de mesure mesurée et de la deuxième valeur de mesure mesurée.

12. Système suivant la revendication 10 ou 11, dans lequel le boîtier (10) de stimulateur cardiaque a une couche extérieure de carbone, la valeur de mesure, de préférence le courant, étant mesurée entre la couche de carbone et l'électrode (6) de bague.
